# EUROPEAN PATENT SPECIFICATION

(11) **EP 3 047 035 B1**
(45) Date of publication and mention of the grant of the patent: **06.02.2019**
(21) Application number: 14781260.6
(22) Date of filing: 17.09.2014
(51) Int. Cl.: C12Q 1/68, G01N 33/50

(54) **BIOMARKERS FOR TUBERCULOSIS**
BIOMARKER FÜR TUBERKULOSE
BIOMARQUEURS POUR LA TUBERCULOSE

(30) Priority: 17.09.2013 GB 201316524
(43) Date of publication of application: 27.07.2016
(73) Proprietor: United Kingdom Research and Innovation, Swindon SN2 1FL (GB)
(72) Inventor: SUTHERLAND, Jayne, Banjul (GM)
(74) Representative: D Young & Co LLP
(86) International application number: PCT/GB2014/052809
(87) International publication number: WO 2015/040377

(56) References cited:
- J. ARKO-MENSAH ET AL: "Increased levels of immunological markers in the respiratory tract but not in serum correlate with active pulmonary mycobacterial infection in mice", CLINICAL MICROBIOLOGY AND INFECTION, vol. 15, no. 8, 1 August 2009 (2009-08-01) , pages 777-786, XP055154916, ISSN: 1198-743X, DOI: 10.1111/j.1469-0691.2009.02734.x
- ZEEV T HANDZEL ET AL: "Increased Th1 and Th2 Type Cytokine Production in Patients with Active Tuberculosis", INTERNET CITATION, 1 June 2007 (2007-06-01), pages 479-483, XP008153329, ISSN: 1565-1088
- JAYNE S. SUTHERLAND ET AL: "Highly Accurate Diagnosis of Pleural Tuberculosis by Immunological Analysis of the Pleural Effusion", PLOS ONE, vol. 7, no. 1, 25 January 2012 (2012-01-25), page e30324, XP055154918, DOI: 10.1371/journal.pone.0030324
- M. VERSLUIS ET AL: "Allergen inhalation decreases adenosine receptor expression in sputum and blood of asthma patients", ALLERGY, vol. 63, no. 9, 1 September 2008 (2008-09-01), pages 1186-1194, XP055154921, ISSN: 0105-4538, DOI: 10.1111/j.1398-9995.2008.01735.x
- LI YANG ET AL: "Relationship between the anti-inflammatory properties of salmeterol/fluticasone and the expression of CD4+CD25+Foxp3+ regulatory T cells in COPD", RESPIRATORY RESEARCH, BIOMED CENTRAL LTD., LONDON, GB, vol. 12, no. 1, 28 October 2011 (2011-10-28), page 142, XP021111472, ISSN: 1465-9921, DOI: 10.1186/1465-9921-12-142
- ROBERTA CASSANO ET AL: "Respirable rifampicin-based microspheres containing isoniazid for tuberculosis treatment", JOURNAL OF BIOMEDICAL MATERIALS RESEARCH PART A, vol. 100A, no. 2, 8 February 2012 (2012-02-08), pages 536-542, XP055154977, ISSN: 1549-3296, DOI: 10.1002/jbm.a.33302
- ARAJ G F ET AL: "Improved detection of mycobacterial antigens in clinical specimens by combined enzyme-linked immunosorbent assays", DIAGNOSTIC MICROBIOLOGY AND INFECTIOUS DISEASES, ELSEVIER SCIENCE PUBLISHING CO., AMSTERDAM, NL, vol. 17, no. 2, 1 August 1993 (1993-08-01) , pages 119-127, XP023915457, ISSN: 0732-8893, DOI: 10.1016/0732-8893(93)90022-Y [retrieved on 1993-08-01]

## Description

### FIELD OF THE INVENTION

The present invention relates to the field of biomarkers of disease. In one aspect, the invention relates to methods for detecting or diagnosing tuberculosis in a subject using such biomarkers.

### BACKGROUND TO THE INVENTION

Tuberculosis is a major public health problem in developing countries due to overcrowding, poor infrastructure and high rates of HIV infection [1]. One of the major roadblocks in reducing TB transmission is the lack of accurate diagnostic tests for use in primary health clinics, which see the majority of TB patients (60%) yet cannot provide laboratory-confirmed diagnosis of TB [2]. Without timely and accurate diagnosis, transmission occurs at a rate of 15 close contacts per year per patient [3].

Current TB diagnostic tests require detection of the bacteria in sputum samples. However, these show considerable variation in sensitivity and specificity, particularly for HIV-positive subjects due to unproductive or paucibacillary sputum. The current gold-standard diagnostic test, sputum culture, is time-consuming, expensive, is prone to contamination and requires infrastructure. New molecular-based tests such as the GeneXpert® provide rapid detection of TB and Rifampicin-resistance in well-equipped laboratories, but are expensive, require infrastructure and lack sensitivity in smear-negative (including children and HIV-positive) subjects.

Rapid tests based on microfluidics (lateral flow tests) hold great promise for TB diagnostics. They are easy to use, cheap, provide an answer within minutes, do not require specialized equipment and are stable at room temperature; making them ideal for use in high-TB burden, resource-poor settings. Lateral flow tests detect markers within a sample of body fluid; urine and blood being the most common. To date, however, no such test has been developed for TB due to lack of sensitivity related to the markers and/or sample type.

The low sensitivity of current blood-based IFN-y release assays (IGRAs) [4] may be due to the migration of TB-specific cells from the blood to the lung during active TB, since significantly higher levels of cellular and soluble host immune markers are present in the pleural fluid compared to blood of the same subjects [5]. Analysis resulted in 96% correct classification of TB or other respiratory diseases regardless of HIV status [5]. Furthermore, analysis did not require antigen-stimulation, with high levels of markers present immediately ex-vivo. Mtb antigens vary considerably according to the stage of infection suggesting that an antigen-independent test would increase specificity.

Despite the reliance on sputum sample collection for TB diagnosis by microbiology, the diagnostic potential of the soluble fraction (i.e. host biomarkers) has not been evaluated, as seen for other respiratory illnesses such as Asthma [6], Cystic Fibrosis [7] and chronic obstructive pulmonary disease (COPD) [7].

There is therefore still a need for improved methods for detecting tuberculosis in subjects. In particular, there is a need for a method which is accurate but rapid, inexpensive and suitable for use at the point of care (i.e. in a non-laboratory setting).

Arko-Mensah et al. (Clinical Microbiology and Infection, 2009, vol. 15, no. 8; XP055154916) describe that increased levels of immunological markers in the respiratory tract but not in serum correlate with active pulmonary mycobacterial infection in mice.

Handzel et al. (Allergy and Clinical Immunology, 2007, vol. 9, pages 479-483; XP008153329) describe increased Th1 and Th2 cytokine production in patients with active tuberculosis.

Sutherland et al. (PLoS One, 2012, vol. 7, issue 1, e30324; XP055154918) describe the diagnosis of pleural tuberculosis by immunological analysis of the pleural effusion.

Versluis et al. (Allergy, 2008, vol. 63, pages 1186-1194; XP055154921) describe that allergen inhalation decreases adenosine receptor expression in sputum and blood of asthma patients.

Yang et al. (Respiratory Research, 2011, vol. 12, page 142; XP021111472) describe the relationship between the anti-inflammatory properties of salmeterol/fluticasone and the expression of CD4⁺CD25⁺Foxp3⁺ regulatory T cells in COPD.

Cassano et al. (J Biomed Mater Res Part A, 2012:100A:536-542; XP055154977) describe respirable rifampicin-based microspheres containing isoniazid for tuberculosis treatment.

### SUMMARY OF THE INVENTION

The invention is defined by the appended claims. Accordingly, the present invention provides a method for detecting tuberculosis in a subject, comprising (a) determining a level of one or more host immune system protein biomarkers in a sputum sample obtained from the subject, wherein the biomarkers are selected from interleukin-1 receptor antagonist (IL-1Ra), interleukin-10 (IL-10), interleukin-13 (IL-13), interleukin-15 (IL-15), fibroblast growth factor (FGF), granulocyte colony stimulating factor (G-CSF), and vascular endothelial growth factor (VEGF); and (b) comparing the levels of the biomarkers in the sputum sample to one or more reference values; wherein a decreased level of IL-1Ra, IL-10, IL-13, IL-15, G-CSF and/or VEGF in the sputum sample compared to the reference value(s), and/or an increased level of FGF in the sputum sample compared to the reference value, is indicative of the presence of tuberculosis in the subject.

In one embodiment, the biomarkers comprise one or more cytokines selected from interleukin-1 receptor antagonist (IL-1Ra), interleukin-15 (IL-15), granulocyte colony stimulating factor (G-CSF) and vascular endothelial growth factor (VEGF). A decreased level of IL-1Ra, IL-15, G-CSF and/or VEGF compared to the reference values is indicative of the presence of tuberculosis in the subject.

In another embodiment, the biomarker comprises fibroblast growth factor (FGF). An increased level of FGF compared to the reference value is indicative of the presence of tuberculosis in the subject.

In another embodiment, the biomarkers comprise IL-13, FGF and IFN-y.

In one embodiment, the subject is suspected to be suffering from a lung disease (or respiratory disorder), and the subject shows one or more symptoms selected from chronic cough, chest pain and fever.

As disclosed herein, the biomarker levels in the sputum sample are preferably compared to corresponding biomarker levels (for each particular biomarker) in control samples. In one embodiment, the control samples may include subjects who are suffering from other lung diseases (or respiratory disorders), such as e.g. pneumonia.

In one embodiment, the levels of the biomarkers are determined by an immunoassay (e.g. each biomarker is detected using an antibody or fragment thereof). Preferably the biomarker levels are determined using an ELISA assay. In one embodiment, detection is performed using a lateral flow immunoassay. In another embodiment, biomarker levels are detected using a multiplex cytokine assay, e.g. using Luminex™ microspheres.

In a further aspect, the present invention provides an anti-tuberculosis agent for use in treating tuberculosis in a subject, wherein the presence of tuberculosis in the subject has been determined by a method as defined as defined above.

Preferably the anti-tuberculosis agent comprises isoniazid, rifampicin, ethambutol and/or pyrazinamide to the subject. In another
preferred embodiment, the treatment for tuberculosis is administered for at least 2 months, at least 4 months, or at least 6 months.

In a further aspect, the present invention provides the use of a lateral flow immunoassay device for detecting tuberculosis in a subject, wherein the device is used to determine the level of one or more host immune system protein biomarkers in a sputum sample obtained from the subject, wherein the device comprises one or more reagents suitable for detecting the one or more host immune system protein biomarkers in a sputum sample obtained from the subject, and wherein the biomarkers are selected from IL-1Ra, IL-10, IL-13, IL-15, FGF, G-CSF and VEGF.

In one embodiment, the device comprises one or more antibodies which bind specifically to one or more of the host immune system biomarkers. Preferably the antibodies bind to IL-13, FGF and/or IFN-y.

In one embodiment, the device comprises a labelled antibody (e.g. an antibody labelled with a detectable marker moiety, such as a fluorescent label or radiolabel) and an immobilized antibody (e.g. an antibody which is immobilized on a solid phase), wherein the labelled and immobilized antibodies each bind to a different epitope on the biomarker, i.e. such that the antibodies do not compete for binding to the biomarker. Thus the labelled and immobilized antibodies are typically capable of binding simultaneously to the biomarker.

Preferably the immobilized antibody is immobilized on a chromatographic carrier material. The chromatographic carrier material is typically a capillary active material, e.g. which permits migration of the fluid component of the sputum sample.

In a preferred embodiment, the device is in the form of a test strip or dipstick, e.g. a chromatographic test strip. Contacting the sample with the test strip may, in one embodiment, permit migration of the liquid in the sample towards the immobilized antibody. In some embodiments, the labelled antibody is deposited on the chromatographic carrier material, and preferably also migrates towards the immobilized antibody following addition of the sample to the test strip. In a preferred embodiment, the labelled antibody and biomarker form a complex which is captured by the immobilized antibody at a test region of the chromatographic strip. The presence of tuberculosis in the subject is preferably indicated by a visible signal (e.g. a colour change) at a test region of the device after contacting the device with the sputum sample.

### BRIEF DESCRIPTION OF THE DRAWINGS

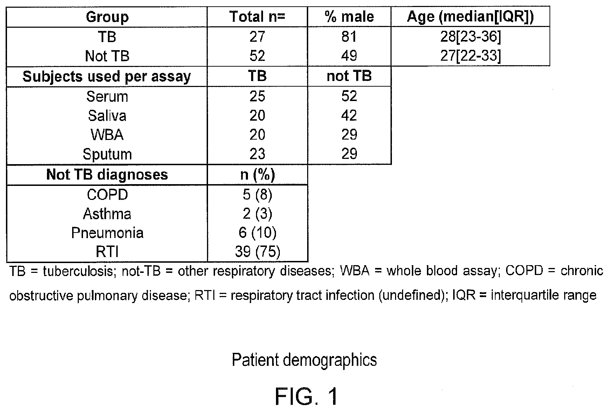

**Figure 1****: Patient demographics.** TB = tuberculosis; not-TB = other respiratory diseases; WBA = whole blood assay; COPD = chronic obstructive pulmonary disease; RTI = respiratory tract infection (undefined); IQR = interquartile range.

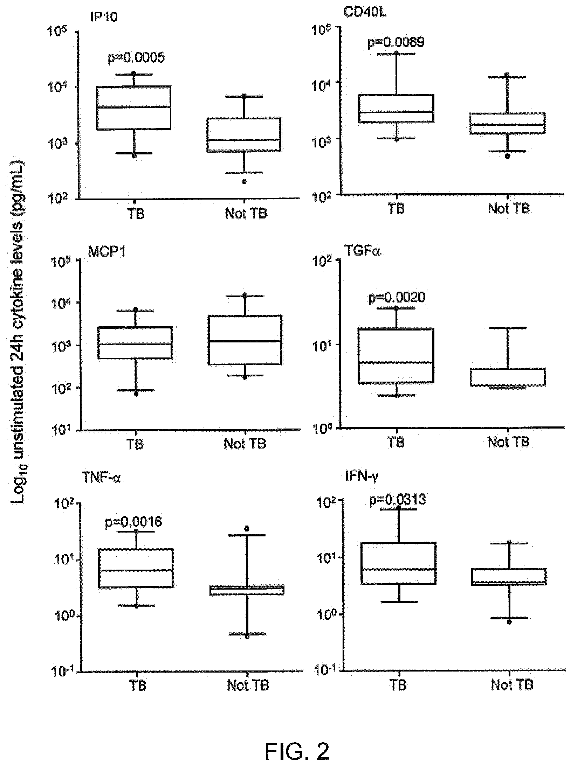

**Figure 2****: Cytokine levels following 24 hour incubation without antigen stimulation (Nil control).** Analysis of 20 TB and 26 non-TB (other respiratory disorders) for cytokine levels following 24 hours incubation. Box indicates interquartile range; line indicates median; bars indicate 5-95% range and dots indicate outliers. Data were anlysed using Mann-Whitney U-test for comparison of TB and non-TB. P-values ≤0.035 were considered significant and are indicated.

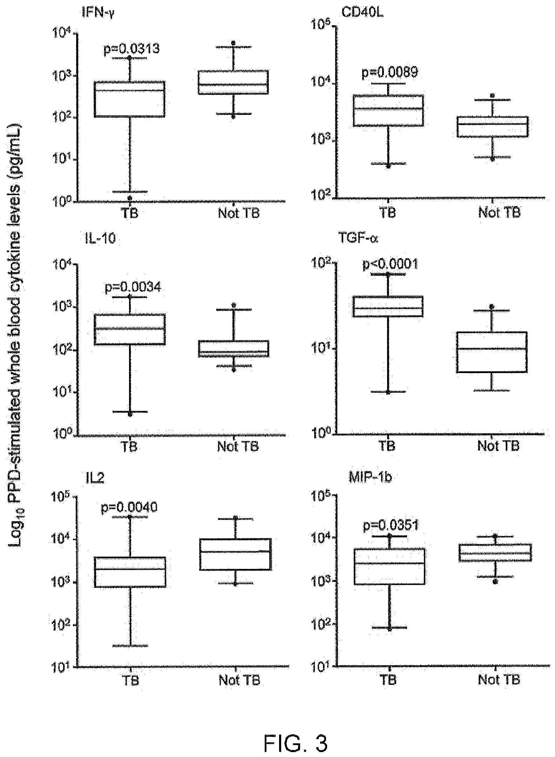

**Figure 3****: Cytokine levels following 24 hour incubation with PPD.** Analysis of 20 TB and 26 non-TB (other respiratory disorders) for cytokine levels following 24 hours incubation with PPD. Box indicates interquartile range; line indicates median; bars indicate 5-95% range and dots indicate outliers. Data were anlysed using Mann-Whitney U-test for comparison of TB and non-TB. P-values ≤0.035 were considered significant and are indicated.

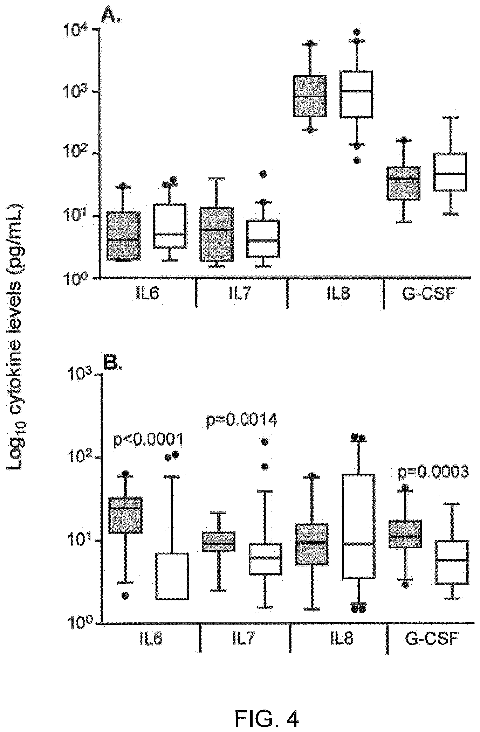

**Figure 4****: Cytokine levels in ex vivo serum and saliva.** A: Analysis of ex vivo saliva from 20 TB (grey) and 42 non-TB (white) subjects. B: Analysis of ex vivo serum from 25 TB (grey) and 52 non-TB (white) subjects. Box indicates interquartile range; line indicates median; bars indicate 5-95% range and dots indicate outliers. Data were anlysed using Mann-Whitney U-test for comparison of TB and non-TB. P-values ≤0.035 were considered significant and are indicated.

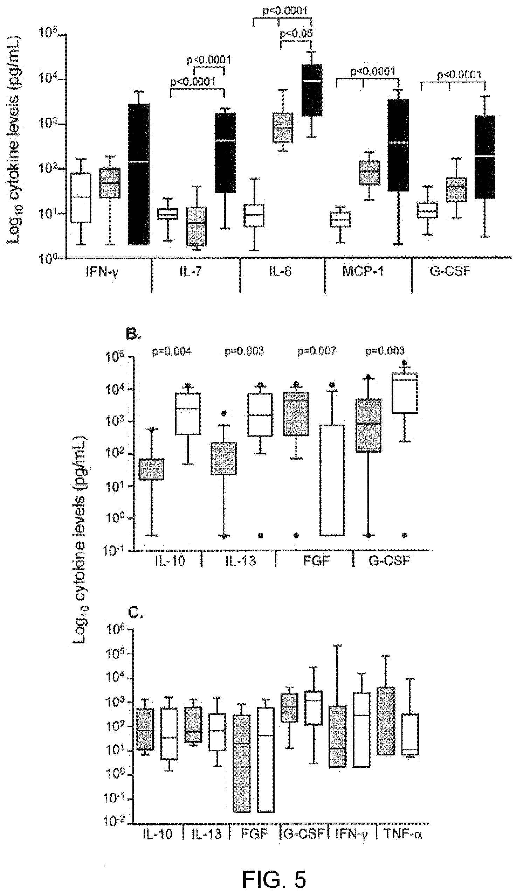

**Figure 5****: Sputum shows high levels of cytokines immediately ex vivo. A**: Comparison of ctyokine levels in serum (white), saliva (grey) and sputum (black) from TB patients (n=25, 20 and 23 respectively). Note that values shown are not adjusted for dilution of the sputum cytokines during digestion. B: Analysis of ex vivo cytokine levels from sputum of TB (n=23) and non-TB (n=29) subjects. Box indicates interquartile range; line indicates median; bars indicate 5-95% range and dots indicate outliers. Data were anlysed using Mann-Whitney U-test for comparison of TB and non-TB. P-values ≤0.035 were considered significant and are indicated.

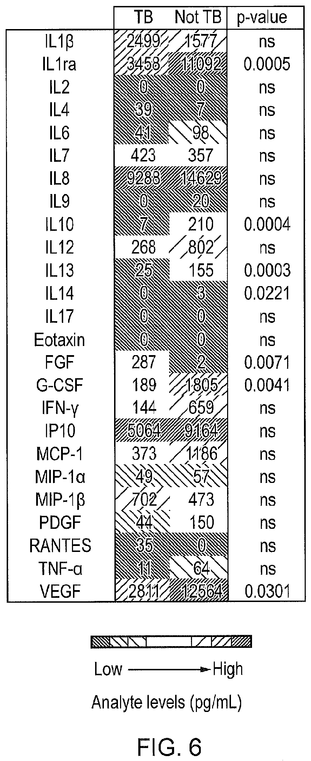

**Figure 6****: Heat map of cytokine levels in *ex vivo* sputum.** Median values are indicated (red = high, blue = low) for subjects with TB (n=23) and those with other respiratory disorders (not-TB; n=29).

### DETAILED DESCRIPTION OF THE INVENTION

The present invention provides a novel method for detecting tuberculosis in a subject. Rather than attempting to detect tuberculosis antigens (which are dynamic and change depending on the state of the bacteria, the amount present, the strain and the virulence), the method advantageously uses host biomarkers which provide a protein signature specific to tuberculosis. This signature is not affected by the strain of mycobacterium underlying the infection, which makes the method more widely applicable than existing antigen-based methods. Moreover, the method can be used to distinguish tuberculosis from other lung conditions such as pneumonia. Because the method is performed on sputum samples but without requiring antigen stimulation or culture, the method is rapid and can be performed in a non-laboratory setting. Thus the method may be performed without the use of needles or blood sampling, without requiring advanced diagnostic techniques, and without needing infrastructure such as medical facilities, electricity and so on. This is particularly important in facilitating the use of the method in developing countries.

### Detecting tuberculosis

In one aspect, the present invention provides a method for detecting tuberculosis in a subject. By "detecting tuberculosis" it is typically meant that the method may be used to determine whether a subject is suffering from tuberculosis. Thus in particular embodiments, the method may be used, for diagnosing tuberculosis; screening a patient population for the presence of tuberculosis; detecting an active tuberculosis infection; detecting the presence of a mycobacterial (e.g. *Mycobacterium tuberculosis*) infection of the lungs; and/or monitoring progression of a tuberculosis infection in a subject.

Tuberculosis (TB) is a chronic, infectious disease that is generally caused by infection with a mycobacterium such as *Mycobacterium tuberculosis.* In one embodiment, detecting TB means detecting an infection by a bacterium of the *Mycobacterium tuberculosis* complex. The *Mycobacterium tuberculosis* complex consists of *M*. *tuberculosis sensu stricto, M. africanum, M. Beijing* and others. Other species of mycobacterium which may be associated with tuberculosis in some cases include *Mycobacterium bovis, Mycobacterium canetti* and *Mycobacterium microti.* Only about 10% of subjects infected with such mycobacteria typically develop active (i.e. symptomatic) tuberculosis. Active tuberculosis infection usually affects predominantly the lungs, resulting in symptoms such as chest pain, fever and a chronic cough producing sputum. Extrapulmonary symptoms may also occur, for instance in the central nervous and lymphatic systems. Thus the present method is typically used to detect an active tuberculosis infection, e.g. in which the subject shows one or more of the above symptoms.

The present method may also be used to distinguish tuberculosis from other lung diseases or respiratory disorders, particularly pneumonia. The method may also be used to distinguish tuberculosis from non-infectious lung diseases, such as chronic obstructive pulmonary disease (COPD) and asthma.

Pneumonia is an inflammatory condition of the lung, typically caused by infection with viruses or bacteria. Symptoms of pneumonia may also include a cough, weight loss, chest pain and fever. Thus in many cases it is difficult to distinguish pneumonia from tuberculosis without performing an X-ray on the subject. However, pneumonia and tuberculosis typically require treatment with quite distinct therapeutic regimens, and the consequences of misdiagnosis can be very serious. For instance, as well as reduced therapeutic efficacy for the individual subject, inaccurate diagnosis may lead to increased disease transmission and increased drug resistance over time. Described herein is an improved method for determining whether a subject suspected to be suffering from a lung disease (e.g. showing one or more symptoms indicative of tuberculosis and/or pneumonia) is suffering from tuberculosis or another lung disease, such as pneumonia.

### Subject

In one embodiment, the subject is a human. However, the method of the present invention is not limited to humans, and may also be performed on e.g. non-human mammals. In a preferred embodiment the subject is an adult human, although in some embodiments the method may be performed on a child or infant.

Typically the subject is suspected to be suffering from a lung disease. Thus the subject may show one or more symptoms associated with lung disease, e.g. a chronic cough (typically with production of sputum), chest pain, difficulty breathing, fever and/or weight loss.

### Sample

In the present invention, the biomarkers are detected in a sputum sample obtained from the subject. Sputum (or phlegm) is thick, viscous fluid derived from the lungs. The sputum sample is typically brought up from the lungs by coughing. Sputum is to be distinguished from saliva, which is considerably thinner and is derived from the mouth rather than the lungs.

Subjects suspected to be suffering from a lung disease such as tuberculosis typically produce significant amounts of sputum which can be analysed using the method described herein. Protocols for obtaining sputum samples from subjects are well known. For instance, in some cases a subject may be instructed to take one or two deep breaths and then cough until they are able to expectorate a thick, viscous sputum sample.

If necessary, further steps can be taken to assist the subject in providing a sputum sample. Breathing hot moist air may thin the mucus of the airway passages, rendering it easier to cough up a sputum sample. For instance, a subject may breathe mist (e.g. provided by a jet hand-held nebulizer or an ultrasonic nebulizer) comprising a 3-15% salt solution for 5-15 minutes before coughing.

### Host immune system biomarkers

In the present invention, one or more host immune system biomarkers selected from IL-1Ra, IL-10, IL-13, IL-15, FGF, G-CSF, and VEGF are detected. By "host biomarkers" it is meant that the biomarkers are derived from the subject itself (rather than e.g. a pathogen which has infected the subject). For example, the host biomarkers may be encoded by the subject's genome rather than the genetic material of an infectious agent. Typically the host biomarkers are therefore human protein biomarkers.

By "immune system biomarkers" it is typically meant that the biomarkers are expressed in the immune system of the subject. For instance, the biomarkers may be expressed by cells of the immune system (e.g. leukocytes such as lymphocytes, neutrophils or macrophages), or the biomarkers may exert a biological effect on cells of the immune system.

Typically the biomarkers are soluble proteins or peptides. For instance, the biomarkers may be signalling molecules secreted by cells of the immune system, and/or which bind to cell surface receptors on cells of the immune system.

Biomarkers described herein include cytokines, chemokines and growth factors. Cytokines are a group of signalling molecules (usually proteins or peptides) which typically have immunomodulatory effects, often by binding to receptors on cells of the immune system (e.g. leukocytes). Examples of sub-groups of cytokines include lymphokines, interleukins, and interferons.

Helper T cell responses are commonly classified as Th1 or Th2, with Th1 being classically associated with cell-mediated (e.g. cytotoxic T cell and macrophage) responses against intracellular pathogens and Th2 with humoral (e.g. secreted antibody production by B cells) responses against extracellular pathogens. Th1 and Th2 responses are typically associated with particular cytokines, which can be classified accordingly.

Th2 cytokines described herein include interleukin-4, interleukin-10, and interleukin-13. In the method of the invention, the biomarkers include IL-10 and IL-13. A decreased level of IL-10 or IL-13 compared to the reference values is typically indicative of the presence of tuberculosis in the subject.

As demonstrated in the Examples the Th1 cytokine IFN-y has diagnostic potential when used in combination with further biomarkers.

In addition to those mentioned above, various other cytokines are used in the present invention. These cytokines include interleukin-1 receptor antagonist (IL-1Ra), interleukin-15, vascular endothelial growth factor (VEGF) and granulocyte colony stimulating factor (G-CSF). A decreased level of such cytokines compared to the reference values is typically indicative of the presence of tuberculosis in the subject.

Chemokines are signalling molecules which mediate chemoattraction (chemotaxis) between cells. Chemokines are typically responsible for recruiting cells such as leukocytes (e.g. neutrophils, monocytes/macrophages or lymphocytes) to sites of inflammation, for instance by binding to cell surface receptors on such cells. Chemokines are commonly soluble proteins or peptides and may in some cases be classed as a sub-group of cytokines. Examples of chemokines described herein include monocyte chemotactic protein-1 (MCP-1), macrophage inflammatory protein-la (MIP-1α), macrophage inflammatory protein-1β (MIP-1β), interferon gamma-induced protein 10 (IP-10), RANTES (Regulated on Activation, Normal T cell Expressed and Secreted) and eotaxin.

Growth factors are signalling molecules which are typically capable of stimulating cellular growth, proliferation and/or differentiation, including angiogenesis. Some agents which are classed as cytokines may also be considered to be growth factors and vice versa, for example VEGF, G-CSF and GM-CSF. Growth factors are commonly proteins or peptides or steroids. In the present invention, the biomarkers comprise fibroblast growth factor (FGF). Typically an increased level of such a growth factor in the sputum sample compared to the reference value is indicative of the presence of tuberculosis in the subject.

### Biomarker combinations

In preferred embodiments, levels of a plurality of (e.g. 2, 3, 4, 5, 6 or more) the host immune system markers are determined in the sputum sample.

In a particularly preferred embodiment, the biomarkers are selected from the group consisting of IL-1Ra, IL-10, IL-13, IL-15, FGF, G-CSF, VEGF and/or IFN-y. More preferably, the biomarkers comprise IL-13, FGF and/or IFN-γ. For instance, in specific embodiments the biomarkers may comprise (i) IL-13 and FGF; (ii) IL-13 and IFN-γ; (iii) FGF and IFN-γ; or (iv) IL-13, FGF and IFN-γ.

### Determining biomarker levels

In the present method, biomarker levels are determined in a sputum sample from the subject. The amount of a particular biomarker in the sample may be measured by any suitable method. For example, methods for detecting protein biomarkers may include the use of an antibody, capture molecule, receptor, or fragment thereof which selectively binds to the protein. Antibodies which bind to the biomarkers described herein are known or may be produced by methods known in the art, including immunization of an animal and collection of serum (to produce polyclonal antibodies) or spleen cells (to produce hybridomas by fusion with immortalised cell lines leading to monoclonal antibodies). The amino acid sequences of the biomarkers described herein are known and available from publicly-accessible databases, and can be used to generate suitable immunogens for antibody production. Detection molecules such as antibodies may optionally be bound to a solid support such as, for example, a plastic surface or beads or in an array. Suitable test formats for detecting protein levels include, but are not limited to, an immunoassay such as an enzyme-linked immunosorbent
assay (ELISA), radioimmunoassay (RIA), Western blotting, antibody arrays, multiplex cytokine assays and immunoprecipitation.

In a preferred embodiment, the biomarkers may be detected using a multiplex cytokine assay, e.g. using Luminex™ microspheres. For instance, antibodies which bind specifically to each cytokine biomarker may be attached to microspheres, e.g. to Luminex™ microspheres designed for use with a Luminex™ Instrument. A large number (e.g. up to 100) different types of microspheres can be mixed and analyzed together. In one embodiment the method is carried out in a single reaction vessel. Each population of microspheres can be distinguished by its unique fluorescence signature or colour.

In a typical multiplex cytokine assay format, different bead types comprise antibodies to different cytokine biomarkers. An aliquot of beads is allowed to incubate with a small volume of test sputum sample. The beads are then washed to remove unbound sample. A detection antibody conjugated to a detectable marker (e.g. biotin, detectable by addition of streptavidin-phycoerythrin) is then added. The sample is then analyzed, e.g. in a flow analyser, by separation of the different bead types and detecting for presence of the marker. The signal intensity from each bead is compared to the signal intensity of a negative control bead included in the bead preparation to determine if the bead is positive or negative for each cytokine.

In another embodiment, the biomarkers may be detected using an antibody array. An antibody array typically comprises an array of immunoglobulin molecules or functional derivatives or equivalents thereof immobilized to discrete regions of a solid support, such that different discrete regions have specificity for different biomarkers. The binding pattern of the immobilized immunoglobulins to their respective antigens is indicative of the presence of particular biomarkers in the sample. Suitable antibody arrays are disclosed, for example, in Chang (1983) J. Immunol. Methods 65, 217 223 and WO00/39580.

Alternatively the level of the biomarker protein may be determined by mass spectroscopy. Mass spectroscopy allows detection and quantification of an analyte by virtue of its molecular weight. Any suitable ionization method in the field of mass spectroscopy known in the art can be employed, including but not limited to electron impact (EI), chemical ionization (CI), field ionization (FDI), electrospray ionization (ESI), laser desorption ionization (LDI), matrix assisted laser desorption ionization (MALDI) and surface enhanced laser desorption ionization (SELDI). Any suitable mass spectrometry detection method may be employed, for example quadrapole mass spectroscopy (QMS), fourier transform mass spectroscopy (FT-MS) and time-of-flight mass spectroscopy (TOF-MS).

### Lateral flow immunoassays

In a particularly preferred embodiment, the level of the biomarker is detected using a lateral flow immunoassay. Lateral flow immunoassays are particularly suited to single-step, point-of care testing (POCT) and provide a sensitive and rapid means for detection of target molecules. Lateral flow immunoassays may be used in sandwich or competitive test formats. Generally high molecular weight analytes with several available epitopes may be analyzed in a sandwich format, whereas smaller molecules representing only a single available epitope may be detected by means of a competitive assay.

Suitable lateral flow immunoassay devices are disclosed, for example, in US 2005/0175992 and US 2007/0059682. Typically a lateral flow device may be in the form of a test strip or dipstick, which is dipped into the sample. The device may be formed from a chromatographic carrier material, such that the liquid in the sample migrates laterally from an application zone towards a reagent zone. Typically molecules of the analyte then encounter a labelled antibody specific for the analyte, and form an analyte-antibody complex. This complex then continues to migrate further laterally towards a test line zone, at which (e.g. in a sandwich assay format) a further antibody is immobilized on the chromatographic carrier material. This second (immobilized) antibody typically binds to a different epitope on the analyte compared to the first (labelled) antibody. The presence of the analyte in the sample is thereby detected by visualization of a signal (such as a colour change) at the test line zone, due to capture of the labelled antibody-analyte complex by the immobilized antibody. In some cases, a further antibody which binds to the labelled antibody in both the presence and absence of analyte (e.g. an anti-immunoglobulin antibody, which binds to the Fc regions of the labelled antibody) may be immobilized at a control line zone which is also present on the test strip. If the test functions correctly, a signal should be visualized at the control line zone whether or not the analyte is present in the sample.

In alternative embodiments, the biomarkers may be detected by a competitive lateral flow immunoassay. In such an assay format, the sample typically first encounters labelled analyte or an analogue thereof (instead of a labelled antibody as in the sandwich assay format discussed above). Analyte derived from the sample then migrates together with the labelled analyte towards the test line, where antibodies to the analyte are immobilized. Unlabelled analyte in the sample competes with the labelled analyte for binding to the antibody, such that the absence of a visible band at the test line is indicative of the presence of the analyte in the sample. This assay format may be employed for instance where it is desired to provide a positive visual signal as indicative of the presence of TB, e.g. where the biomarker is a Th2 cytokine, which is decreased in sputum samples from subjects with the disease.

### Antibodies

The detection methods described herein preferably use one or more antibodies which bind to the host immune system biomarkers described herein. Suitable antibodies are commercially available or may be generated using known techniques.

Antibodies comprise immunoglobulin molecules. Immunoglobulin molecules are in the broadest sense members of the immunoglobulin superfamily, a family of polypeptides comprising the immunoglobulin fold characteristic of antibody molecules, which contains two β sheets and, usually, a conserved disulphide bond. Antibodies, as used herein, refers to complete antibodies or antibody fragments capable of binding to a selected target biomarker, and including Fv, ScFv, F(ab') and F(ab')₂, monoclonal and polyclonal antibodies, engineered antibodies including chimeric, CDR-grafted and humanised antibodies, and artificially selected antibodies produced using phage display or alternative techniques.

Antibodies may be obtained from animal serum, or, in the case of monoclonal antibodies or fragments thereof, produced in cell culture. Recombinant DNA technology may be used to produce the antibodies according to established procedure, in bacterial, yeast, insect or preferably mammalian cell culture. The selected cell culture system preferably secretes the antibody product.

Growing of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, for example foetal calf serum, or trace elements and growth sustaining supplements, for example feeder cells such as normal mouse peritoneal exudate cells, spleen cells, bone marrow macrophages, 2-aminoethanol, insulin, transferrin, low density lipoprotein, oleic acid, or the like. The culture medium may be serum-free or animal-produce free, such as a chemically defined medium, in order to minimise animal derived contamination. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

Insect cells may be cultured in serum free medium, which is cheaper and safer compared to serum containing medium. Recombinant baculovirus may be used as an expression vector, and the construct used to transfect a host cell line, which may be any of a number of lepidopteran cell lines, in particular *Spodoptera frugiperda Sf9,* as known in the art. Reviews of expression of recombinant proteins in insect host cells are provided by Altmann et al. (1999), Glycoconj J 1999, 16, 109-23 and Kost and Condreay (1999), Curr Opin Biotechnol, 10, 428-33.

*In vitro* production provides relatively pure antibody preparations and allows scale-up to give large amounts of the desired antibodies. Techniques for bacterial cell, yeast, insect and mammalian cell cultivation are known in the art and include homogeneous suspension culture, for example in an airlift reactor or in a continuous stirrer reactor, or immobilised or entrapped cell culture, for example in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired target by immunoblotting, by an enzyme immunoassay, for example a sandwich assay or a dot-assay, or a radioimmunoassay.

For isolation of the antibodies, the immunoglobulins in the culture supernatants or in the ascitic fluid may be concentrated, for example by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or immunoaffinity chromatography, for example affinity chromatography with the a protein containing a target or with Protein-A.

Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

The methods described here may employ recombinant nucleic acids comprising an insert coding for a heavy chain variable domain and/or for a light chain variable domain of antibodies. By definition such nucleic acids comprise coding single stranded nucleic acids, double stranded nucleic acids consisting of the coding nucleic acids and of complementary nucleic acids thereto, or these complementary (single stranded) nucleic acids themselves.

Antibodies may moreover be generated by mutagenesis of antibody genes to produce artificial repertoires of antibodies. This technique allows the preparation of antibody libraries; antibody libraries are also available commercially. Hence, artificial repertoires of immunoglobulins, preferably artificial ScFv repertoires, can be used as an immunoglobulin source.

Isolated or cloned antibodies may be linked to other molecules, for example nucleic acid or protein association means by chemical coupling, using protocols known in the art (for example, Harlow and Lane, Antibodies: a Laboratory Manual, (1988) Cold Spring Harbor, and Maniatis, T., Fritsch, E. F. and Sambrook, J. (1991), Molecular Cloning: A Laboratory Manual. Cold Spring Harbor, New York, Cold Spring Harbor Laboratory Press). Such methods may be used to produce labelled antibodies or to immobilize the antibody on a solid phase.

In some embodiments (e.g. involving lateral flow immunoassays) the antibody may be labelled. Typically a labelled antibody is capable of producing a detectable signal. The signal may be, for example, the generation of an enzymatic activity, such as protease activity, transcriptional activity or luminescence inducing activity. Preferably, however, the signal is emission or absorption of electromagnetic radiation, for example, light. More preferably the signal is a visible signal, e.g. the signal is detectable with the naked eye. The signal may be, for example, a colour change which takes place when the labelled antibody is present.

Methods of conjugating visible or fluorescent labels to various entities, including peptides, polypeptides and antibodies, are well known in the art. In certain embodiments, it may be desirable to include spacing means between the antibody and the label. The spacing means may comprise linkers or spacers which are polymers of differing lengths (the length of which may be controlled by controlling the degree of polymerisation). Numerous spacers and linkers are known in the art, and the skilled person will know how to choose and use these, depending on the application. The skilled person will also know what spacer length to use.

### Comparison to reference values

In the present invention, the levels of the biomarkers in the sputum sample are compared to one or more reference values. The reference value may be, for example, a predetermined measurement of a level of the biomarker which is present in a sputum sample from a normal subject, i.e. a subject who is not suffering from tuberculosis. In some embodiments, the reference value may be derived from a subject (or a population of subjects) who is suffering from a lung disease other than tuberculosis, e.g. pneumonia. The reference value may, for example, be based on a mean or median level of the biomarker in a control population of subjects, e.g. 5, 10, 100, 1000 or more subjects (who may either be age- and/or gender-matched or unmatched to the test subject) who show no symptoms of tuberculosis. Preferably the level of the biomarker in the test sample differs by at least 1%, 5%, at least 10%, at least 20%, at least 30%, or at least 50% compared to the control value.

The control value may be determined using corresponding methods to the determination of lipid levels in the test sample, e.g. using one or more samples taken from a control population of subjects. For instance, in some embodiments biomarker levels in control samples may be determined in parallel assays to the test samples. In alternative embodiments, the control value may have been previously determined, or may be calculated or extrapolated, without having to perform a corresponding determination on a control sample with respect to each test sample obtained.

In the case of lateral flow assays, the presence or absence of the biomarker in the sample may typically be determined by the presence or absence of a visible signal (e.g. a colour change) at the test line on the lateral flow device, i.e. the result can normally be determined by the naked eye. It will be recognised by the skilled person that lateral flow devices can also be used to determine whether a level of a particular biomarker is above or below a particular cut-off value (which may correspond to the reference value as described herein). Cut-off and reference values may generally be determined using various statistical techniques, including Receiver-Operator Curve analysis, as described in the examples below.

For instance, the assay arrangement and conditions and the relative amounts of labelled, immobilized and control antibodies may be selected such that the strength of the visual signal at the test line can be compared to the visual signal at the control line in order to provide an indication of whether the level of the biomarker is above or below the reference value. Alternatively, the lateral flow test may be performed on a sample from the subject in parallel with a separate (control) test on a sample from a subject known to be not suffering from tuberculosis. The control may also be a sample not derived from a patient but containing a defined amount of the biomarker. In either case, comparing the results from the test strip to the control strip may provide an indication of the levels of the biomarker in the sputum sample from the subject compared to the reference value.

In another alternative embodiment, the signal on the lateral flow device may be quantified to provide a more accurate indication of biomarker levels. For instance, the intensity of the signal at the test line may be determined in order to quantify the amount of analyte in the sample. Handheld diagnostic devices such as lateral flow readers may be used, e.g. to illuminate the test line and measure a specific wavelength of light indicative of the label. Image processing algorithms may be incorporated in such readers in order to correlate the signal with analyte concentrations.

### Treating lung disease

The present disclosure also describes a method of treating a subject suspected to be suffering from a lung disease or respiratory disorder. Typically the method comprises a step of performing a detection method as described above, and treating the subject based on the results thereof. In particular, if the levels of biomarkers in the sputum sample are indicative of the presence of tuberculosis in the subject, the method typically involves a step of administering a therapeutically effective amount of an anti-tuberculosis treatment (e.g. comprising one or more therapeutic agents) to the subject. Typically such a treatment for TB is continued for an extended period of time, e.g. at least 1 month, at least 2 months, at least 3 months, at least 4 months or at least 6 months.

If the levels of biomarkers in the sputum sample are indicative of the absence of tuberculosis in the subject, the method may involve a step of administering an alternative therapy for lung disease to the subject. The alternative therapy may be a treatment for e.g. pneumonia, asthma or chronic obstructive pulmonary disease. For example, an anti-pneumonia therapeutic agent may be administered to the subject. Commonly such treatments for lung diseases other than TB may be more short-term than for TB treatments. Thus in such embodiments, the treatment may be continued for up to 1 week, up to 2 weeks, up to 3 weeks or up to 1 month.

Therapeutic agents and protocols useful for treating tuberculosis are well known to a skilled person. For instance, the anti-tuberculosis therapy may comprise administration of one or more agents selected from isoniazid, rifampicin, ethambutol and/or pyrazinamide. Typically active TB infection is treated using two or more therapeutic agents in combination. The treatment is preferably administered for at least 6 months, although this may be divided into an initial intensive treatment period followed by an extended continuation period. Thus the standard short course treatment for active TB infection is isoniazid, rifampicin, pyrazinamide, and ethambutol for two months, followed by isoniazid and rifampicin alone for a further four months. In the case of failure of such a first line therapy, a second line therapy may in some cases be used. Second line therapies may include aminoglycosides (e.g. amikacin, kanamycin); fluoroquinolones (e.g. ciprofloxacin, levofloxacin, moxifloxacin); thioamides (e.g. ethionamide, prothionamide); cycloserine (e.g., closerin); terizidone, capreomycin, viomycin or enviomycin.

In contrast, therapeutic agents used for treating pneumonia (particularly bacterial pneumonia) are commonly broad-spectrum antibiotics. For instance, suitable antibiotic agents include amoxicillin, doxycycline, clarithromycin, macrolides (such as azithromycin or erythromycin). In some cases cephalosporins, carbapenems and vancomycin may also be used, e.g. given intravenously and used in combination particularly in the case of hospital-acquired infections. Typically the treatment may be administered for e.g. 3 to 5 days, 7 to 10 days or up to 2 weeks.

The therapeutic agent may be administered to a subject using a variety of techniques. For example, the agent may be administered systemically, which includes by injection including intramuscularly or intravenously, orally, sublingually, transdermally, subcutaneously, or internasally. Preferably the agent is administered orally. The concentration and amount of the therapeutic agent to be administered will typically vary, depending on the nature of the disease, the type of agent that is administered, the mode of administration, and the age and health of the subject.

The therapeutic agent may be formulated in a pharmaceutical composition in e.g. solid or tablet form or in liquid form, e.g. together with a pharmaceutically acceptable diluent. The compositions may routinely contain pharmaceutically acceptable amounts of diluents, excipients and other suitable carriers. Appropriate carriers and formulations are described, for example, in Remington's Pharmaceutical Sciences (Remington's Pharmaceutical Sciences, Mack Publishing Company, Easton, Pa., USA 1985).

### Kits

The present invention also describes a kit suitable for performing the method as described above. In particular, the kit may comprise reagents suitable for detecting the biomarkers described above, e.g. one or more host immune system biomarkers, or a biomarker combination as defined herein. Typically the reagents may comprise antibodies which bind specifically to the
biomarkers, or a combination of biomarkers as defined herein. For instance the kit may comprise one, two, three or four different antibodies, each of which binds to a different biomarker selected from those defined above.

Such kits may optionally further comprise one or more additional components, e.g. reagents suitable for performing an ELISA assay using antibodies which bind to the biomarkers. For instance, the kits may comprise capture and detection antibodies for each biomarker, secondary antibodies, detection reagents, solid phases (e.g.reaction plates or beads), standards (e.g. known concentrations of each biomarker in the form of recombinant proteins) as well as buffers suitable for performing any step of an ELISA method. The kits may further comprise vials, containers and other packaging materials for storing the above reagents, as well as instructions for performing a method as defined herein.

The kit may be in the form of, or comprise, a lateral flow immunoassay device. Such a device may comprise one or more antibodies which bind specifically to a host immune system biomarker, or a combination of biomarkers, as described herein. Suitable antibodies are commercially available or may be generated using known techniques.

The invention will now be described by way of example only with respect to the following specific embodiments.

### EXAMPLES

In the present study several sample types were analysed to determine the optimal fluid and biomarker combination for diagnosis of TB. Sputum host biomarkers were significantly higher than levels in antigen-stimulated blood and resulted in 96% correct classification of TB, regardless of smear or HIV status. Thus the present invention provides a test that allows 'laboratory-free' detection of TB. The benefits of this are clear: with 10 million new diagnoses every year, but only 16% confirmed by laboratory, the ability to rapidly detect TB will considerably reduce the burden of TB.

### Methods

### Subjects:

Subjects were consecutively recruited from the outpatient clinic and ward at the Medical Research Council Unit, Fajara, The Gambia. All subjects were adults (≥18 years) presenting with a cough that had lasted more than 2 weeks plus one other clinical symptom (ie weight loss, fever) suggestive of TB. Exclusion criteria included previous treatment for TB or co-morbidities such as malaria. Following written informed consent all subjects had full hematological, microbiological, biochemical and symptoms evaluation. HIV testing was performed and sputum, saliva, serum, and heparinised blood samples were collected for immunological evaluations. Clinical symptoms (questionnaire, physical examination), chest x-ray, and microbiology (sputum smear and culture) were used to classify patients into two groups: those with culture-confirmed TB and those with other respiratory diseases (**Figure 1**). Sputum culture was performed using liquid culture (BACTEC™, Becton-Dickinson, USA) and presence of *Mycobacterium tuberculosis* complex (MTBC) was confirmed using Capilia rapid TB tests (Taun Laboratories, Japan). Ethical approval was obtained from the Gambian government/MRC joint ethics committee.

### Microbiological confirmation

Sputum samples were analysed by Ziehl-Nielsen (ZN) stain using LED microscopy. An aliquot was decontaminated and cultured (BACTEC™, Becton-Dickinson, USA). Positive cultures were confirmed by Capilia rapid TB test (Tauns, Japan) and stored in glycerol at -70°C.

### Spoligotyping

Stored isolates were grown in Middlebrook 7H9 broth with OADC (oleic acid, albumin, dextrose, and catalase) supplement for DNA extraction. 10ng of DNA was used for spoligotype analysis with commercially available membranes (Isogen Biosciences, The Netherlands). Spoligotype films were scanned and classified using software designed in Matlab (Mathworks, USA), followed by manual editing and confirmation. Each spoligotype pattern was classified into a binary code and the result was entered in a Microsoft Access database (Redmond, USA). For isolates that could not be reliably classified as M. tuberculosis or M. africanum based on spoligotype analysis alone, the presence or absence of lineage defining large sequence polymorphisms RD702 and TbD1 was assessed, as previously described.

### Multi-plex cytokine assays

### Sample preparations

Serum and saliva were aliquoted and frozen at -20°C until needed. Sputum was digested for 15 minutes at room temperature with 0.1% Dithiothreitol (DTT). An equal volume of phosphate-buffered saline (PBS) was added, the samples centrifuged (600_{gmax}, 5 min) and supernatant collected and stored at -20°C. For heparinised blood, we used 450µl of undiluted blood per well of a 24-well plate. Blood was stimulated with purified protein derivative (PPD) (10µg/mL; Statens Serum Institute, Denmark), ESAT-6/CFP-10 (EC; 10µ/mL) and two dormancy antigens, Rv0081 and Rv2029 (both at 10µg/mL). After 24h incubation (37°C, 5% CO₂), supernatants were harvested and stored at - 20°C prior to analysis.

### Multi-plex analysis for cytokine production

Samples were analysed using either a custom 13-plex (stimulated blood) or 27-plex Bio-Plex (serum, saliva and sputum) pre-mixed cytokine/chemokine kits according to the manufacturer's instructions (Bio-Rad, Belgium). Following pre-wetting of the filter plate, 50µl of bead suspension was added to each well and washed twice. 50µl of samples and standards were then added and incubated for 1 hour at 300rpm. The plate was washed 3 times then 50µl of detection antibody added and the plate incubated for 30 min. at 300rpm. After washing, 25µl of streptavidin-PE was added to each well and incubated for 10 min. The plate was again washed and resuspended in 125µl of assay buffer, sealed, mixed and immediately read on the Bio-plex analyser using Bioplex manager software (version 4.0). A quality control was used for each plate to control for inter-assay variation.

### Statistical analysis

Value of analytes measured from the antigen-specific samples (whole blood assay only) had background subtracted (unstimulated results). Saliva, serum and sputum values were all derived from unstimulated samples and therefore did not require background subtraction. TB and non-TB subjects were compared using Mann-Whitney U-test, Logistic Regression and Receiver-operator curve analyses were performed and adjusted for age and sex. Matched sputum and antigen-stimulated cultures were analysed using Friedman test followed by Dunn's multiple comparisons test. Graphs were generated using Graphpad Prism version 6.0 (Software MacKiev, USA) and statistical analysis with SPSSv20 (IBM, USA). P values ≤0.035 were considered significant to account for false-discovery rates (FDR).

### Results

### Subject demographics

In total we analysed 52 non-TB and 27 confirmed TB subjects (Figure 1). There was no difference in the median age between the TB and non-TB subjects, but the TB group had a significantly higher proportion of males compared to the non-TB group (81% compared to 49%). This fits with the epidemiology of TB in The Gambia and as such all results were adjusted for sex and age. Of the non-TB group, final diagnoses were based on clinical evaluation and response to treatment with 13/52 subjects diagnosed with Chronic Obstructive Pulmonary Disease (COPD; n=5), Asthma (n=2), Pneumonia (n=6) and the remainder grouped into general respiratory tract infections (RTI; no confirmed diagnosis). Numbers of subjects varied for each sample type due to sample availability (Figure 1): 20 TB and 26 non-TB subjects were used for whole blood antigen (WBA)-stimulation analysis; 25 TB and 52 non-TB subjects for serum analysis; 20 TB and 42 non-TB subjects for saliva analysis, and 23 TB and 29 non-TB subjects for sputum analysis.

### Classification of TB using whole blood 24-hour antigen-stimulated culture supernatants

We analysed cytokine profiles of TB and non-TB subjects following overnight stimulation with Nil (no antigen), EC, PPD, Rv0081 and Rv2029. Profiles were compared for Antigen-stimulated (Ag), Ag-Nil and Nil for each cytokine. IP10 and MCP-1 levels were high in both groups following all stimulations (mean values 7522pg/mL and 6547pg/mL respectively) whilst TGF-α, EGF and VEGF were low (mean values 9pg/mL, 26pg/mL and 23pg/mL respectively). There were a number of analytes that were significantly higher in subjects with confirmed TB compared to non-TB in Nil cultures including IP10, CD40L, TGF-α, TNF-α and IFN-γ (p=0.0005, p=0.0089, p=0.0020, p=0.0016 and p=0.0313 respectively; **Fig. 2**). Following antigen stimulation, the majority of differences were observed prior to background subtraction with levels of CD40L and TGF-α significantly higher in TB compared to non-TB subjects regardless of antigen used. Following background subtraction, the majority of differences were observed in the PPD-stimulated cultures with higher levels of CD40L, IL-10 and TGF-α in TB compared to non-TB subjects (p=0.0089, p=0.0034 and p<0.0001 respectively) but lower levels of IFN-y, IL-2 and MIP-1β (p=0.0313, p=0.0040 and p=0.0351 respectively; **Fig. 3**). We performed logistic regression analyses to determine the optimal antigen-biomarker combination for diagnosis of TB: following PPD stimulation, the best classifier was TGF-α with an AUC of 0.86 [95%CI 0.73-1.0], sensitivity of 96.2% [95%CI 80.4- 99.9], specificity of 80.0% [95%CI 56.3-94.3] and a likelihood ratio of 4.8. Following EC stimulation, levels of TGF-α resulted in 84.6% sensitivity [95%CI 65.1-95.6] and 80% specificity [95%CI 56.3-94.3]. The best classification was achieved following PPD stimulation with a combination of CD40L, TGF-α and IL10 giving 89% correct classification of TB or not-TB (data not shown). Importantly, there was no difference in the host immune profiles for TB subjects infected with different Mtb strains (*M*. *tuberculosis* or *M*. *africanum*) following stimulation with any of the antigens (data not shown).

### Analysis of ex vivo saliva and plasma cytokine levels:

A major factor for an effective diagnostic test in developing countries is time to diagnosis. We therefore wanted to assess body fluids for cytokine levels immediately *ex vivo.* Saliva showed higher levels of cytokines than serum but no difference was seen between TB and non-TB subjects for any cytokine analysed (**Fig. 4A**). Serum had relatively low levels of all cytokines with MIP-1β detected at the highest level (86pg/mL) in TB subjects. However, there were significantly higher levels of IL-6, IL7 and G-CSF in serum from TB compared to non-TB subjects (p<0.0001, p=0.0014 and p=0.0003 respectively; **Fig. 4B**).

### Sputum shows high levels of cytokines immediately ex vivo:

We analysed the soluble fraction of digested sputum for ex vivo cytokine levels and found surprisingly high levels without requiring antigen stimulation compared to both saliva and serum. We performed cross-sectional analysis of ex vivo cytokine levels from subjects confirmed to have TB (**Fig. 5A**). Levels of IL-4, IL-5, IL-10, IL-13, IL-7, IL-8, IL-12(p70) and MIP-1β were all significantly higher in sputum compared to both saliva and serum (illustrated in **Fig. 5A** by IL-7 and IL-8) while IL-1β, IL-17, G-CSF, GM-CSF, MCP-1 and TNF-α were significantly higher in both saliva and sputum compared to serum (illustrated in **Fig. 5A** by G-CSF and MCP-1). IL-6 was the only cytokine lower in saliva compared to both serum (p<0.01) and sputum (p<0.0001) with no difference between serum and sputum (data not shown), with no difference in IFN-y levels seen between the three sample types (**Fig. 5A**).

We next compared cytokine levels in sputum from TB and non-TB subjects (**Fig. 6** and **Fig. 5B**). Interestingly, we found no significant difference in pro-inflammatory cytokines (i.e. TNF-α, IFN-y, IP-10; data not shown) but significantly lower levels of IL-10 (p=0.004), IL-13 (p=0.003) and IL-15 (p=0.022) was found in sputum from TB compared to non-TB subjects (**Fig. 6** and **Fig. 5B**). Additionally, innate cytokines IL-1Ra, G-CSF and VEGF were all significantly lower (p=0.005, p=0.004, p=0.030 respectively), whilst FGF was significantly higher in TB compared to non-TB subjects (median 287 pg/mL in TB compared to 2.2 pg/mL in non-TB subjects; p=0.007; **Fig. 6** and **Fig. 5B**). Levels of FGF alone gave 74% correct classification (sensitivity 78% [95%CI 56-93] and specificity 67% [95%Cl 47-83]) of TB. Logistic regression showed a combination of IL-13, FGF and IFN-γ gave 96% correct classification of TB and 85% of non-TB (overall 90%). Importantly, no difference was observed in subjects with HIV co-infection as seen previously with pleural fluid analysis [5].

Microbiological confirmation of TB is performed on 2-3 samples per patient, with samples varying in time of collection (3 samples within 24h is standard at MRC). There is variation in smear results with different samples and therefore we wanted to determine cytokine levels in multiple sputum samples obtained from the same person (n=15). We found no difference in the levels of any cytokine analysed (**Fig. 5C**).

### Discussion

Alongside high sensitivity and specificity, an important criteria for development of a lateral-flow based point-of-care test for TB is time to results. Loss to follow-up or defaulting from care is a major problem in healthcare delivery in resource-poor settings; it is therefore imperative that patients can be diagnosed and receive appropriate care within hours of undergoing TB testing [2]. The current diagnostic tests based on blood-derived host immune factors require at least 24 hours of sample processing and even then levels are often too low for further development of a rapid test. To the best of our knowledge, we are the first group to examine levels of cytokines in sputum as a new tool for TB diagnostics. We found extremely high levels of cytokines in *ex vivo* sputum with minimal sample preparation require; a combination of FGF, IL-13 and IFN-γ resulted in 96% correct classification of TB in subjects presenting with similar symptoms (i.e. cough duration >2 weeks). These findings enable the development of a rapid point-of-care test for TB.

We have previously shown that sensitivity and specificity for TB were significantly increased by analysing samples from the site of infection compared to the blood [5]. This is due to the migration of effector CD4+ T cells to the lung from the blood during TB disease resulting in a virtual absence of these cells in the blood but a predominant and highly activated population in the lung [5]. These cells are responsible for producing IFN-γ and other cytokines required to fight the infection and thus directly correlate with the increased levels of soluble factors present in lung-derived samples compared to blood. Sputum is routinely used for microbiological detection of Mtb. It is easily obtainable and cytokine levels do not appear to be affected by HIV co-infection making it an ideal sample type for development of a lateral flow test for TB. In addition, there was no influence of strain of infection and reproducibility was high with different sputum samples from the same subject.

Levels of IL-4, IL-5, IL-10, IL-13, IL-7, IL-8 and MIP-1b were all significantly higher in sputum compared to both saliva and serum while IL-1b, IL-17, G-CSF, GM-CSF, MCP-1 and TNF-α were all significantly lower in serum compared to saliva and sputum, with no difference between saliva and sputum. These findings illustrate the difference in immune subsets that will be responding locally compared to the periphery. For instance, increased levels of innate and Th17 cytokines were seen in saliva and sputum compared to blood, indicating increase mucosal-associated immunity at these sites. Interestingly, no difference in Th1 cytokine levels were observed between the ex vivo sample types and there was also no significant difference in IFN-γ, IP-10 and TNF-α levels in *ex vivo* sputum from TB compared to subjects with other respiratory disorders. Conversely, the Th2 cytokines, IL-10 and IL-13 were both significantly lower in TB compared to non-TB; indicating a bias towards Th1 responses in subjects with TB, which could result in increased immune pathology. G-CSF is required for neutrophil recruitment and was found to be significantly lower in sputum from TB compared to non-TB subjects. This is interesting since neutrophils are a major component in the protective immune response to TB [8] and G-CSF administration has been shown to increase response to TB therapy [9]. While most factors were lower in TB compared to non-TB, FGF was significantly higher. The fibroblast growth factor (FGF) signalling pathway is integral to the pathogenesis of many airway diseases and in the growth and development of the normal lung [10]. Interestingly, Mtb infected fibroblasts lose their capacity for antigen presentation, suggesting that Mtb may evade T helper immune surveillance by infecting fibroblasts, thereby resulting in bacterial persistence [11].

The diagnostic development requirements determined suggested by FIND (Foundation for Innovative New Diagnostics) include at least 75% and ideally >95% sensitivity (including 100% of culture positive samples). We only analysed subjects with culture-confirmed TB and of these, only 3 were smear negative (14%) so it is difficult at this stage to determine sensitivity in smear negative subjects. However, 96% correct classification of TB using a combination of FGF, IL-13 and IFN-y from sputum is significantly higher than results reported from current blood, breath or urine based tests. We limited our analysis to 27 cytokines/chemokines but with increased analytes available for detection, markers that allow >95% specificity and sensitivity are likely to be determined. In conclusion, we have shown the use of sputum rather than blood significantly increases diagnostic accuracy of immune-based TB tests and reduces time to results. These findings hold promise for future development of a rapid lateral-flow based diagnostic test for TB that is applicable for use in resource-limited settings.

### Summary

Tuberculosis (TB) is a significant public health problem in developing countries with 9 million new cases and 1.4 million deaths each year. A major roadblock in reducing the TB burden is the absence of a fast and accurate diagnostic test for use in health clinics with minimal infrastructure. We analysed samples from patients presenting with symptoms suggestive of TB but prior to confirmation. Following clinical and microbiological evaluation they were subsequently diagnosed with TB or other respiratory diseases (non-TB). We evaluated host biomarkers in sputum, saliva, serum and whole blood antigen-stimulated cultures to determine the optimal sample type and biomarker combination for diagnosis of TB. Overnight stimulation of whole blood with ESAT-6/CFP-10 (EC) or PPD generated high levels of cytokines; following PPD stimulation, the best classifier was TGF-α with an AUC of 0.86 [95%Cl 0.73-1.0], sensitivity of 96.2% [95%Cl 80.4- 99.9], specificity of 80.0% [95%Cl 56.3-94.3] and a likelihood ratio of 4.8. Following EC stimulation, levels of TGF-α resulted in 84.6% sensitivity [95%CI 65.1-95.6] and 80% specificity [95%CI 56.3-94.3]. The best classification was achieved following PPD stimulation with a combination of CD40L, TGF-α and IL10 giving 89% correct classification of TB or not-TB. However, 24 hours of stimulation is not ideal for a 'rapid' diagnostic test. *Ex vivo* saliva had significantly higher levels of cytokines compared to *ex vivo* serum, but could not discriminate between patient groups. Serum levels of IL7, IL-8 and G-CSF were all significantly higher in TB compared to non-TB subjects (p<0.0001, p=0.0014 and p=0.0003 respectively). We also analysed cytokine levels in the soluble fraction of sputum; which is used for routine microbiology and is obtainable from the majority of adult pulmonary TB patients. No significant difference in pro-inflammatory cytokine levels (IFN-γ, IP-10, TNF-α) was seen, but significantly lower levels of Th2 cytokines (IL-10 (p=0.0004), IL-13 (p=0.0003) and IL-15 (p=0.0221) and innate cytokines (IL-1ra (p=0.0005), VEGF (0.0301) and G-CSF (p=0.0041)) and higher FGF (p=0.007) was seen in sputum from TB compared to non-TB subjects. A combination of IL-13, FGF and IFN-y resulted in 96% correct classification of TB and 85% of non-TB (overall 90%) regardless of HIV status. The present invention thus provides a rapid, point-of-care test for TB.

### References:

1. WHO Global Tuberculosis Report 2012. WHO fact sheet number 104. Available at: http://www.who.int/tb/publications/factsheet_global.pdf.
2. Batz H-G, Cook GS, Reid SD. Towards lab-free TB diagnostics. WHO-TDR 2011. Accessed at: http://www.stoptb.org/wg/tb_hiv/assets/documents/MSF_Stop%20TB_Imperial_TAG_TowardsLabF reeTBDX_July%2011_Web2.pdf
3. WHO tuberculosis fact sheet number 104. March, 2012. Accessed at http://www.who.int/mediacentre/factsheets/fs104/en/
4. WHO diagnostics evaluation series. Laboratory-based evaluation of 19 commercially available rapid diagnostic tests for tuberculosis. 2008. Accessed at: http://www.who.int/tdr/publications/documents/diagnostic-evaluation-2.pdf
5. Sutherland JS, Garba D, Fombah AE, Mendy-Gomez A, Mendy FS, Antonio M, Townend J, Ideh RC, Corrah T, Ota MO. Highly accurate diagnosis of pleural tuberculosis by immunological analysis of the pleural effusion. PLoS One 2012;7:e30324.
6. Dente FL, Carnevali S, Bartoli ML, Cianchetti S, Bacci E, Di Franco A, Vagaggini B, Paggiaro P. Profiles of proinflammatory cytokines in sputum from different groups of severe asthmatic patients. Ann Allergy Asthma Immunol 2006;97:312-320.
7. Eickmeier O, Huebner M, Herrmann E, Zissler U, Rosewich M, Baer PC, Buhl R, Schmitt-Grohé S, Zielen S, Schubert R. Sputum biomarker profiles in cystic fibrosis (CF) and chronic obstructive pulmonary disease (COPD) and association between pulmonary function. Cytokine 2010;50:152-157
8. Yang CT, Cambier CJ, Davis JM, Hall CJ, Crosier PS, Ramakrishnan L. Neutrophils exert protection in the early tuberculous granuloma by oxidative killing of mycobacteria phagocytosed from infected macrophages. Cell Host Microbe 2012;12:301-312.
9. Cormican U, Schey S, Milburn HJ. G-CSF enables completion of tuberculosis therapy associated with iatrogenic neutropenia. Eur Respir J 2004;23:649-650.
10. Dosanjh A. The fibroblast growth factor pathway and its role in the pathogenesis of lung disease. J Interferon Cytokine Res 2012;32:111-114.
11. Mariotti S, Sargentini V, Pardini M, Giannoni F, De Spirito M, Gagliardi MC, Greco E, Teloni R, Fraziano M, Nisini R. Mycobacterium tuberculosis may escape helper T cell recognition by infecting human fibroblasts. Hum Immunol 2013;74:722-729.

## Claims

1. A method for detecting tuberculosis in a subject, comprising:
(a) determining a level of one or more host immune system protein biomarkers in a sputum sample obtained from the subject, wherein the biomarkers are selected from interleukin-1 receptor antagonist (IL-1 Ra), interleukin-10 (IL-10), interleukin-13 (IL-13), interleukin-15 (IL-15), fibroblast growth factor (FGF), granulocyte colony stimulating factor (G-CSF), and vascular endothelial growth factor (VEGF); and
(b) comparing the levels of the biomarkers in the sputum sample to one or more reference values, said one or more reference values comprising a level of the biomarker in a sputum sample from a subject who is not suffering from tuberculosis; wherein a decreased level of IL-1Ra, IL-10, IL-13, IL-15, G-CSF and/or VEGF in the sputum sample compared to the reference value(s), and/or an increased level of FGF in the sputum sample compared to the reference value, is indicative of the presence of tuberculosis in the subject.

2. A method according to claim 1, wherein the biomarkers comprise one or more cytokines selected from IL-1Ra, IL-15, G-CSF and VEGF; wherein a decreased level of the biomarker(s) compared to the reference value(s) is indicative of the presence of tuberculosis in the subject.

3. A method according to claim 1, wherein the biomarker comprises FGF, and an increased level of the biomarker compared to the reference value is indicative of the presence of tuberculosis in the subject.

4. A method according to any preceding claim, comprising
(a) determining the levels of the host immune system protein biomarkers IL-13 and FGF and the further host immune system protein biomarker interferon-y (IFN-y) in a sputum sample obtained from the subject; and
(b) comparing the levels of the biomarkers in the sputum sample to one or more reference values;
wherein a decreased level of IL-13 and IFN-γ in the sputum sample compared to the reference values, and an increased level of FGF in the sputum sample compared to the reference value, is indicative of the presence of tuberculosis in the subject.

5. A method according to any preceding claim, wherein the subject is suspected to be suffering from a lung disease, and the subject shows one or more symptoms selected from chronic cough, weight loss and fever.

6. A method according to any preceding claim, wherein the levels of the biomarkers are determined by a lateral flow immunoassay, a multiplex cytokine assay or an antibody array.

7. An anti-tuberculosis agent for use in treating tuberculosis in a subject, wherein the presence of tuberculosis in the subject has been determined by a method as defined in any of claims 1-6.

8. An anti-tuberculosis agent for use according to claim 7, wherein the anti-tuberculosis agent comprises isoniazid, rifampicin, ethambutol and/or pyrazinamide; and/or wherein the treatment for tuberculosis is administered for at least 2 months, at least 4 months, or at least 6 months.

9. Use of a lateral flow immunoassay device for detecting tuberculosis in a subject, wherein the device is used to determine the level of one or more host immune system protein biomarkers in a sputum sample obtained from the subject, wherein the device comprises one or more reagents suitable for detecting the one or more host immune system protein biomarkers in a sputum sample obtained from the subject, and wherein the biomarkers are selected from IL-1Ra, IL-10, IL-13, IL-15, FGF, G-CSF and VEGF.

10. A use according to claim 9, wherein the device comprises one or more antibodies which bind specifically to one or more of the biomarkers.

11. A use according to claim 9, wherein the device comprises one or more reagents suitable for detecting the host immune system protein biomarkers IL-13 and FGF and the further host immune system protein biomarker IFN-γ in a sputum sample obtained from the subject.

12. A use according to any of claims 9 to 11 wherein the device comprises a labelled antibody and an immobilized antibody, the labelled and immobilized antibodies each binding to a different epitope on the biomarker; optionally wherein the immobilized antibody is bound to a chromatographic carrier material.

13. A use according to any of claims 9-12, wherein the device is in the form of a test strip or dipstick; and/or wherein the presence of tuberculosis in the subject is indicated by a visible signal at a test region of the device after contacting the device with the sputum sample.

## Patentansprüche

1. Verfahren zum Nachweisen von Tuberkulose bei einem Individuum, umfassend:
(a) Bestimmen eines Spiegels von einem oder mehr Proteinbiomarkern des Wirtsimmunsystems in einer aus dem Individuum erhaltenen Sputumprobe, wobei die Biomarker ausgewählt sind aus Interleukin-1-Rezeptorantagonist (IL-1Ra), Interleukin-10 (IL-10), Interleukin-13 (IL-13), Interleukin-15 (IL-15), Fibroblastenwachstumsfaktor (FGF), Granulozytenkoloniestimulationsfaktor (G-CSF) und vaskulärem endothelialem Wachstumsfaktor (VEGF); und
(b) Vergleichen der Spiegel der Biomarker in der Sputumprobe mit einem oder mehr Referenzwerten, wobei die ein oder mehr Referenzwerte einen Spiegel des Biomarkers in einer Sputumprobe aus einem Individuum umfassen, das nicht an Tuberkulose leidet;
wobei ein niedrigerer Spiegel von IL-1Ra, IL-10, IL-13, IL-15, G-CSF und/oder VEGF in der Sputumprobe im Vergleich zu dem/den Referenzwert(en) und/oder ein höherer Spiegel von FGF in der Sputumprobe im Vergleich zu dem Referenzwert das Vorhandensein von Tuberkulose in dem Individuum anzeigt.

2. Verfahren nach Anspruch 1, wobei die Biomarker ein oder mehr Cytokine, ausgewählt aus IL-1Ra, IL-15, G-CSF und VEGF umfassen; wobei ein niedriger Spiegel des/der Biomarker(s) im Vergleich zu dem/den Referenzwert(en) das Vorhandensein von Tuberkulose in dem Individuum anzeigt.

3. Verfahren nach Anspruch 1, wobei der Biomarker FGF umfasst und ein höherer Spiegel des Biomarkers im Vergleich zu dem Referenzwert das Vorhandensein von Tuberkulose in dem Individuum anzeigt.

4. Verfahren nach einem vorhergehenden Anspruch, umfassend:
(a) Bestimmen der Spiegel der Proteinbiomarker IL-13 und FGF des Wirtsimmunsystems und des weiteren Proteinbiomarkers Interferon-y (IFN-y) des Wirtsimmunsystems in einer aus dem Individuum erhaltenen Sputumprobe; und
(b) Vergleichen der Spiegel der Biomarker in der Sputumprobe mit einem oder mehr Referenzwerten;
wobei ein niedrigerer Spiegel von IL-13 und IFN-γ in der Sputumprobe im Vergleich zu den Referenzwerten und ein höherer Spiegel von FGF in der Sputumprobe im Vergleich zu dem Referenzwert das Vorhandensein von Tuberkulose in dem Individuum anzeigt.

5. Verfahren nach einem vorhergehenden Anspruch, wobei vermutet wird, dass das Individuum an einer Lungenerkrankung leidet, und das Individuum ein oder mehr Symptome zeigt, die aus chronischem Husten, Gewichtsverlust und Fieber ausgewählt sind.

6. Verfahren nach einem vorhergehenden Anspruch, wobei die Spiegel der Biomarker durch einen Lateral-Flow-Immunassay, einen Multiplex-Cytokin-Assay oder einen Antikörperarray bestimmt werden.

7. Anti-Tuberkulosemittel zur Verwendung bei der Behandlung von Tuberkulose in einem Individuum, wobei das Vorhandensein von Tuberkulose in dem Individuum durch das Verfahren nach einem der Ansprüche 1 bis 6 bestimmt wurde.

8. Anti-Tuberkulosemittel zur Verwendung nach Anspruch 7, wobei das Anti-Tuberkulosemittel Isoniazid, Rifampicin, Ethambutol und/oder Pyrazinamid umfasst; und/oder wobei die Behandlung auf Tuberkulose für mindestens 2 Monate, mindestens 4 Monate oder mindestens 6 Monate verabreicht wird.

9. Verwendung einer Lateral-Flow-Immunassay-Vorrichtung zum Nachweisen von Tuberkulose in einem Individuum, wobei die Vorrichtung zum Bestimmen des Spiegels von einem oder mehr Proteinbiomarkern des Wirtsimmunsystems in einer aus dem Individuum erhaltenen Sputumprobe verwendet wird, wobei die Vorrichtung ein oder mehr Reagenzien umfasst, die zum Nachweisen des einen oder mehr Proteinbiomarkern des Wirtsimmunsystems in einer aus dem Individuum erhaltenen Sputumprobe geeignet ist, und wobei die Biomarker ausgewählt sind aus der Gruppe, bestehend aus IL-1Ra, IL-10, IL-13, IL-15, FGF, G-CSF und VEGF.

10. Verwendung nach Anspruch 9, wobei die Vorrichtung einen oder mehr Antikörper umfasst, der/die spezifisch an einen oder mehr der Biomarker bindet/binden.

11. Verwendung nach Anspruch 9, wobei die Vorrichtung ein oder mehrere Reagenzien umfasst, die zum Nachweisen der Proteinbiomarker IL-13 und FGF des Wirtsimmunsystems und des weiteren Protein-Biomarkers IFN-γ des Wirtsimmunsystems in einer aus dem Individuum erhaltenen Sputumprobe geeignet sind.

12. Verwendung nach einem der Ansprüche 9 bis 11, wobei die Vorrichtung einen markierten Antikörper und einen immobilisierten Antikörper umfasst, wobei die markierten und immobilisierten Antikörper jeweils an ein unterschiedliches Epitop auf dem Biomarker binden, wobei der immobilisierte Antikörper gegebenenfalls an ein Chromatographie-Trägermaterial gebunden ist.

13. Verwendung nach einem der Ansprüche 9 bis 12, wobei die Vorrichtung in der Form eines Teststreifens oder Messstäbchens vorliegt; und/oder wobei das Vorhandensein von Tuberkulose in dem Individuum durch ein sichtbares Signal an einer Testregion der Vorrichtung angezeigt wird, nachdem die Vorrichtung mit der Sputumprobe in Kontakt gekommen ist.

## Revendications

1. Méthode de détection de la tuberculose chez un sujet, comprenant les étapes consistant à :
(a) déterminer un niveau d'un ou de plusieurs biomarqueurs protéiniques du système immunitaire de l'hôte dans un échantillon d'expectoration obtenu auprès du sujet, où les biomarqueurs sont choisis parmi les suivants : antagoniste de récepteur d'interleukine-1 (IL-1Ra), interleukine-10 (IL-10), interleukine-13 (IL-13), interleukine-15 (IL-15), facteur de croissance des fibroblastes (FGF), facteur de stimulation des colonies de granulocytes (G-CSF) et facteur de croissance de l'endothélium vasculaire (VEGF) ; et
(b) comparer les niveaux des biomarqueurs dans l'échantillon d'expectoration à une ou plusieurs valeurs de référence, lesdites une ou plusieurs valeurs de référence comprenant un niveau du biomarqueur dans un échantillon d'expectoration issu d'un sujet qui ne souffre pas de tuberculose ;
où un niveau inférieur de IL-1 Ra, IL-10, IL-13, IL-15, G-CSF et/ou VEGF dans l'échantillon d'expectoration par rapport aux valeurs de référence, et/ou un niveau supérieur de FGF dans l'échantillon d'expectoration par rapport à la valeur de référence, indiquent la présence de la tuberculose chez le sujet.

2. Méthode selon la revendication 1, où les biomarqueurs comprennent une ou plusieurs cytokines choisies parmi IL-1 Ra, IL-15, G-CSF et VEGF ; où un niveau inférieur du ou des biomarqueurs par rapport aux valeurs de référence indique la présence de la tuberculose chez le sujet.

3. Méthode selon la revendication 1, où le biomarqueur comprend FGF, et un niveau supérieur en biomarqueur par rapport à la valeur de référence indique la présence de la tuberculose chez le sujet.

4. Méthode selon l'une quelconque des revendications précédentes, comprenant les étapes consistant à
(a) déterminer les niveaux des biomarqueurs protéiniques du système immunitaire de l'hôte IL-13 et FGF et du biomarqueur protéinique supplémentaire du système immunitaire de l'hôte interféron-γ (IFN-γ) dans un échantillon d'expectoration obtenu auprès du sujet ; et
(b) comparer les niveaux des biomarqueurs dans l'échantillon d'expectoration à une ou plusieurs valeurs de référence ;
où un niveau inférieur de IL-13 et de IFN-γ dans l'échantillon d'expectoration par rapport aux valeurs de référence, et un niveau supérieur de FGF dans l'échantillon d'expectoration par rapport à la valeur de référence indiquent la présence de la tuberculose chez le sujet.

5. Méthode selon l'une quelconque des revendications précédentes, où le sujet est soupçonné de souffrir d'une maladie pulmonaire, et le sujet présente un ou plusieurs symptômes choisis parmi toux chronique, perte de poids et fièvre.

6. Méthode selon l'une quelconque des revendications précédentes, où les niveaux des biomarqueurs sont déterminés par un dosage immunologique à flux latéral, un dosage en multiplex des cytokines ou un array d'anticorps.

7. Agent antituberculeux pour utilisation dans le traitement de la tuberculose chez un sujet, où la présence de la tuberculose chez le sujet a été déterminée par une méthode selon l'une quelconque des revendications 1 à 6.

8. Agent antituberculeux pour utilisation selon la revendication 7, où l'agent antituberculeux comprend isoniazid, rifampicine, éthambutol et/ou pyrazinamide ; et/ou où le traitement de la tuberculose est administré pendant au moins 2 mois, pendant au moins 4 mois ou pendant au moins 6 mois.

9. Utilisation d'un dispositif de dosage immunologique à flux latéral pour la détection de la tuberculose chez un sujet, où le dispositif est utilisé pour déterminer le niveau d'un ou de plusieurs biomarqueurs protéiniques du système immunitaire de l'hôte dans un échantillon d'expectoration obtenu auprès du sujet, où le dispositif comprend un ou plusieurs réactifs adaptés à la détection du ou des biomarqueurs protéiniques du système immunitaire de l'hôte dans un échantillon d'expectoration obtenu auprès du sujet, et où les biomarqueurs sont choisis parmi IL-1Ra, IL-10, IL-13, IL-15, FGF, G-CSF et VEGF.

10. Utilisation selon la revendication 9, où le dispositif comprend un ou plusieurs anticorps qui se lient spécifiquement à un ou plusieurs des biomarqueurs.

11. Utilisation selon la revendication 9, où le dispositif comprend un ou plusieurs réactifs adaptés à la détection des biomarqueurs protéiniques du système immunitaire de l'hôte IL-13 et FGF et du biomarqueur protéinique supplémentaire du système immunitaire de l'hôte IFN-γ dans un échantillon d'expectoration obtenu auprès du sujet.

12. Utilisation selon l'une quelconque des revendications 9 à 11, où le dispositif comprend un anticorps marqué et un anticorps immobilisé, chacun des anticorps marqué et immobilisé se liant à un épitope différent du biomarqueur ; éventuellement, où l'anticorps immobilisé est lié à un matériau de support chromatographique.

13. Utilisation selon l'une quelconque des revendications 9 à 12, où le dispositif se présente sous la forme d'une bandelette d'essai ou d'une bandelette à tremper ; et/ou où la présence de la tuberculose chez le sujet est indiquée par un signal visible au niveau d'une région d'essai du dispositif après mise en contact du dispositif avec l'échantillon d'expectoration.
